# EUROPEAN PATENT SPECIFICATION

(11) **EP 1 959 831 B1**
(45) Date of publication and mention of the grant of the patent: **14.07.2010**
(21) Application number: 06824265.0
(22) Date of filing: 15.11.2006
(51) Int. Cl.: A61B 5/11

(54) **MOTION TRACKING SYSTEM**
BEWEGUNGSVERFOLGUNGSSYSTEM
SYSTEME DE SUIVI DE MOUVEMENT

(30) Priority: 16.11.2005 NL 1030440
(43) Date of publication of application: 27.08.2008
(73) Proprietor: Xsens Technologies B.V., 7521 PR Enschede (NL)
(72) Inventor: SLYCKE, Per Johan, NL-7412 XP Deventer (NL); VELTINK, Petrus Hermanus, NL-7481 CV Haaksbergen (NL); ROETENBERG, Daniel, NL-7524 AX Enschede (NL)
(74) Representative: Hatzmann, Martin
(86) International application number: PCT/NL2006/000572
(87) International publication number: WO 2007/058526

(56) References cited:
- US-A- 5 744 953
- US-B1- 6 361 507
- US-B1- 6 474 159
- US-B1- 6 691 074

## Description

The invention relates to a motion tracking system for tracking an object composed of object parts in a three-dimensional space. In particular, the invention relates to a motion tracking system for tracking the movements of a human body.

Measurement of physical motion with a high resolution is important for many medical, sports and ergonomic applications. Further, in the film and computer game market, there is a great need for motion data for the purpose of advanced animation and special effects. Finally, for instance, motion data is needed in Virtual Reality (VR) and Augmented Reality (AR) applications for training and simulation.

At this moment, there are a number of technologies available for tracking and recording motion data. They generally require that an infrastructure be constructed around the object to be tracked. An example thereof are optical systems which use a large number of cameras, fixedly arranged around the object of which the motion is to be tracked. Therefore, an optical measuring system can only track the motion of the object in the volume which is recorded with the cameras. Further, a camera system suffers from occlusion when the view of the camera of the object is obstructed by another object or it does not work in certain light conditions. Another example of systems which need an infrastructure are systems which can track position and orientation on the basis of generating magnetic fields and detecting the generated field with a magnetometer. The advantage of such magnetic systems is that they do not suffer from occlusion and that they can work in any light condition. However, these types of systems are relatively sensitive to magnetic disturbances. Further, these systems need relatively large transmitters due to the rapid decrease in magnetic field strength (1/d²). A typical transmitter with a range of only 1 m has a dimension of 10x10x10 cm and a weight of 2 kg. Transmitters with a larger range of 3 m have a dimension of 30x30x30 cm.

In many cases, it is desired to measure motion data of body segments in an ambulatory manner, in any place, without dependence on provisions in the environment.

A technology which is very suitable for this makes use of inertial sensors in combination with earth magnetic field sensors. Inertial sensors, such as gyroscopes and accelerometers measure their own motion independently of other systems. The measured gravitational acceleration can be used as a reference system direction. The magnetic field sensors measure the earth magnetic field which is used as a reference for the forward direction in the horizontal plane (north). However, such inertial and magnetic measuring modules (integrated 3-axis acceleration transducers, gyroscopes and magnetometers) can only measure orientations with respect to a fixed earth-bound coordinate system.

It is principally not possible to measure position with such inertial sensors by twice integrating the acceleration, since the starting position is unknown. Further, the error of the position estimation will increase rapidly in time due to integration drift as a result of addition of noise, offset and incorrectly subtracted gravitational acceleration as a result of orientation errors. By combining inertial sensors with, for instance, an ultrasound measuring system or optical measuring systems, it is possible to measure position. However, due to the combination with such systems, the motion tracking system as a whole depends on external systems again, and is therefore not ambulatory.

### Description of prior art

US patent no. 5,744,953, Hansen, 1998, "Magnetic motion tracker with transmitter placed on tracked object" shows a configuration which is intended to be suitable to wear on the human body in an ambulatory manner. The configuration contains magnetic field sensors which detect a magnetic field generated by a transmitter positioned on the body. The motion tracking, in particular the relative motion tracking of the constituent body parts, is therefore done by sampling the magnetic field sensors. The magnetic field sensors are tracked in space by combination with the acceleration transducers, which can mark the position with respect to the ground. In order to obtain sufficient time resolution, it is necessary to make the transmitter transmit magnetic fields at a high frequency. Thus, relatively much power is required so that, in practice, this system has limited uses.

US patent no. 6,691,074 shows a combination of magnetic coil detectors and accelerometers, where the transmitters are not placed on the body. The motion information is derived by the combination of signals of the magnetic coil detectors and the accelerometers, in particular, by means of Kalman filtering. In this manner, it is possible to detect disturbances in the generated magnetic field, something from which magnetic systems always suffer, and the motion tracking becomes less sensitive to magnetic disturbances. The system shown has fixedly arranged transmitters, creating a dependence on provisions in the environment.

US patent no. 5,930,741 discusses the use of faster and slower sensors, including inertial sensors and magnetic sensors. However, the system makes use of external magnetic field transmitters, which are disadvantageous with ambulatory use.

A further embodiment of a motion tracking system is known from US 6 361 507. The known system represents a self-contained sensor apparatus wherein a signal is generated corresponding to at least two of the three orientational aspect of yaw, pitch and roll of a human-scale body, relative to an external frame.

### Summary of the invention

It is an object of the invention to provide a system in which positions of an object, in particular of object parts relative to one another, and orientations of object parts can be measured in any place in an ambulatory manner, without dependence on provisions in the environment. Another object of the invention is to provide a system where the required power is relatively limited so that measuring can be done with the system for an acceptable time using a portable energy source. This object is achieved by means of a system according to claim 1. In particular, the processor is configured to make the transmitters transmit an intermittent magnetic field, where the position and/or orientation information in a period between the intermittent transmitting is derived by means of the motion information coming from the inertial measurement unit, which is periodically calibrated with the motion information coming from the magnetic measuring module.

Thus, at regular intervals, relative position and orientation measurements are carried out with the magnetic field sensor system. This prevents drift in the position estimation based on the acceleration transducers. Further, parameters of a model of the sensors, such as for instance the offset and/or gain of the acceleration transducers, are identified, preferably with the aid of signal-processing methods which enable estimation of stochastic signals, such as Kalman filters. With the aid of the inertial module, orientations can be determined and changes of position, in particular by double integration in time of accelerations after a rotation of the signals to the inertial coordinate system and the subtraction of the gravitational acceleration. Due to this configuration, a relatively low transmitting frequency of the magnetic field transmitters is sufficient, because the output of the magnetic measuring module substantially serves to calibrate the orientation and position information obtained from the inertial module. Thus, the required power can be reduced considerably reducing the weight of the design and so that ambulatory uses can be possible for sufficiently long periods (hours to a day). Further, the system can have a considerably less expensive design than the currently available motion tracking systems.

In addition, the relative positions and orientation estimations can also be improved by making use of anatomical knowledge of the body and typical motion characteristics.

### Brief description of Figures

The invention will be explained in more detail with reference to the Figure, in which:
Fig. 1 shows a schematic view of fusing of measurement data, where a calibration of the inertial sensors is used at times t₁ and t₂;
Fig. 2 shows a schematic representation of a motion tracking system provided on a body to be tracked;
Fig. 3 shows a schematic view of a model representation of the body of Fig. 2;
Fig. 4 shows a schematic view of a motion tracking system with transmitters and receivers mounted on one body part;
Fig. 5 shows a schematic view of the general signal-processing structure;
Fig. 6 shows a further schematic view of the signal-processing structure;
Fig.7 shows a more detailed schematic view of the signal-processing structure; and
Fig. 8 shows a preferred embodiment of a sensor cluster for use in a system according to the invention.

### Description of the invention with reference to the drawings

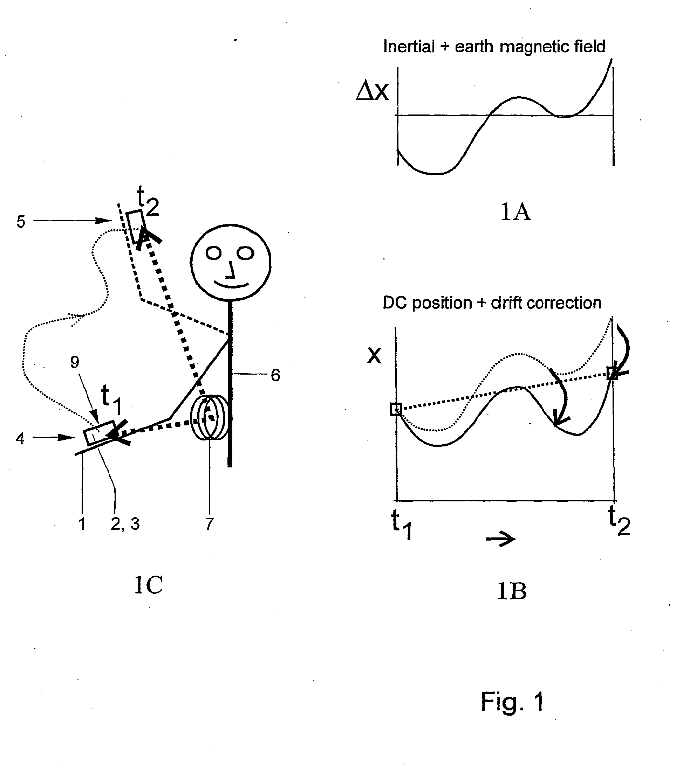

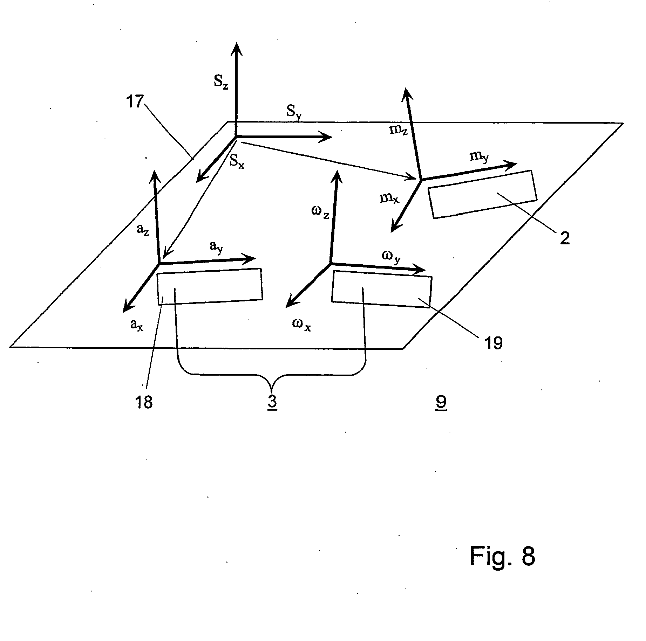

Fig. 1 shows the principle of combining data coming from an inertial measurement unit, periodically calibrated by data coming from a pulsed magnetic sensor system. To this end, illustration C shows a schematic view of a test subject who moves an arm 1 with a magnetic sensor 2 and an inertial measurement unit 3 thereon from a first position 4 to a second position 5 between the times t₁ and t₂. On the trunk 6, a magnetic field transmitter 7 is fixed, typically a current coil for generating a magnetic (dipole) field. At both times, as measurement is carried out with the aid of the magnetic system, which particularly comprises magnetic module 2 and magnetic field transmitters 7. These measurements serve to determine the relative positions and orientations of arm 1 at those times t₁ and t₂ for recalibrating the measurement data coming from the inertial measurement unit 3 (see illustration A). In this manner, the relative positions of a few measuring points provided on the body can be updated by the pulsated magnetic sensor system 2, 7 (see illustration B, in which the measurement data of A are fused with the measurements at times t₁ and t₂). Thus, in the intervening time period, the position and orientation change can be estimated from measurements coming from the inertial measurement unit 3. Also, during the time of magnetic transmission, inertial measurements can be used to track changes in position and orientation of both the transmitter 7 as well as the magnetic measuring module 2, in particular, to compensate for a reception of a signal that may be deformed due to a relative angular velocity, velocity and/or acceleration between receiving sensor cluster 9 and transmitter 7. In a preferred embodiment, the magnetic measuring module and the inertial measurement unit are integrated in a sensor cluster 9. The output of the inertial measurement unit can yield momentary acceleration and orientation data. For this, typically a linear accelerometer can be used in combination with a rate gyroscope, as described in more detail with reference to Fig. 8. By recording the orientation of the linear accelerator with the aid of the gyroscope, the actual free acceleration of the object part can be calculated after subtraction of the gravitational acceleration. To this end, the calculated free acceleration is twice integrated in time, which yields a position value. The associated drift due to the integration and inaccuracies of the inertial module can be corrected by periodical position calibration by means of the magnetic sensor system 2, 7. From this correction, for instance, parameters in the sensor model of the inertial system 3 can be obtained.

It follows from the Figure that the field transmitters 7 can have a transmitting frequency which is lower than a predetermined sampling frequency which is related to a motion resolution realized by the system. This resolution can be realized by means of intermediate measurements which are periodically recalibrated with the above-mentioned magnetic measuring module 2. Typically such a transmitting frequency is lower than 10 Hz.

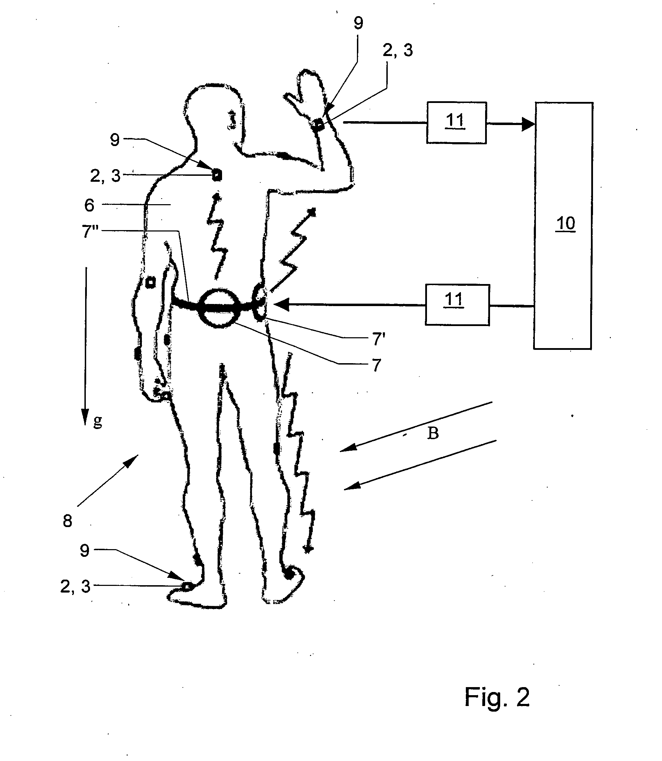

Fig. 2 shows a first embodiment of the invention where a body 8 is shown which is provided with magnetic field transmitters 7. To this end, the transmitters 7 are integrated in clothing and at least one current coil is wound around the trunk 6 for generating a magnetic field. Other body parts may also be utilized to be enclosed by means of a current coil in a well defined manner. Thus, a good fixation of the transmitter with respect to the body part is realized.

In the clothing, further, sensor clusters 9 may be integrated, which comprise an inertial module 3 and a magnetic measuring module 2. These sensor clusters 9 are distributed over multiple body parts so that, of the respective body parts, the relative position with respect to the transmitter can be determined. Of course, the sensor clusters 9 may also be provided on the body with other fixing constrictions, such as a strap or the like. If integrated in the clothing, it is again advantageous if a body part is enclosed by a field transmitter 7, in particular by the coil for transmitting a magnetic field.

Further, the magnetic measuring modules 2 may be sensitive to the earth magnetic field B, which defines a fixed axis in the space. This orientation in the space can further be determined by deriving the gravitational acceleration g by means of the inertial measurement unit 3, in particular the gyroscopes present therein, as is explained with reference to Fig. 8.

Accordingly, in Fig. 2, a number of transmitters 7 are shown with a fixed positional relationship with respect to at least one body part for transmitting respective magnetic fields, with mutually different main directions. In particular, in Fig. 2, three transmitters 7, 7', 7" are shown with mutually different main directions for being able to determine, in three dimensions, a distance and spatial orientation (i.e. six degrees of freedom) with respect to the transmitter of a body part, in particular of a sensor cluster 9 of field receivers 2 provided on the body part for receiving the respective magnetic fields of the transmitters 7. To this end, a processor 10 and required regulating and control circuits 11 are provided for controlling the transmitters 7 and receiving and amplifying the signals received from the field receivers 2 and inertial measurement unit 3. The sensor clusters 9 may further each comprise an inertial measurement unit 3 for recording a linear acceleration and/or angular velocity in three main directions. The circuits 11 are arranged such that they can receive and process the signals coming from the inertial measurement unit 3 and pass them to the processor 10 on in a suitable form. Therefore the processor 10 is programmed for controlling the transmitters 7 and receiving signals coming from the field receivers 2 and the inertial measurement units 3.

Further, the processor 10 comprises a module for deriving position and orientation information of the body parts on the basis of the received signals. To this end, the processor 10 is configured such that the transmitters 7 are controlled intermittently with a particular (not necessarily fixed) time period. As has been explained with reference to Fig. 1, the position and orientation information is derived by the processor during the time period by means of the motion information received from the inertial measurement units 3 and the magnetic field receiver 2 which can measure the earth magnetic field B during the time period. Periodically this information is calibrated with the motion information coming from the magnetic fields of transmitter 7 measured by field receivers 2. Due to information redundancy, the processor can relate the position information with respect to a fixed axis in the space which is derived from the gravitational field g and/or the earth magnetic field B. To this end, advantageous use is made of a stochastic maximum likelihood estimator, such as for instance a Kalman filter or particle filter.

The period between the intermittent transmitting can be determined on the basis of a derived measuring error, so that, for instance, exceeding a particular threshold value thereof can activate a new cycle of orientation and/or position measurement by means of magnetic fields.

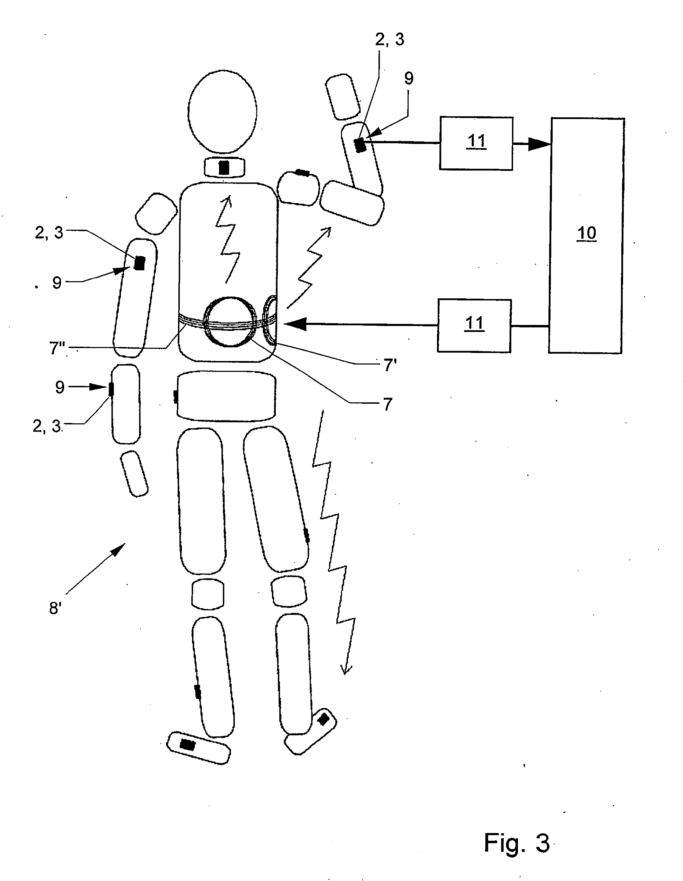

Fig. 3 shows a further view of an object measurement, where it is schematically shown that the object, in this case a human body 8', is modeled on the basis of a number of rigid segments which are movable relative to one another. By relating the received values from the magnetic field receivers 2 and the inertial measurement units 3 to the model, an increased accuracy can be realized with the position and orientation measurement. Under the feet 12 of the person to be tracked, further, a number of sensors can be provided (not shown) for measuring a ground reaction force and torque. In this manner, a still further fixed orientation in the space can be derived and internal forces and torques of the object to be tracked can be derived.

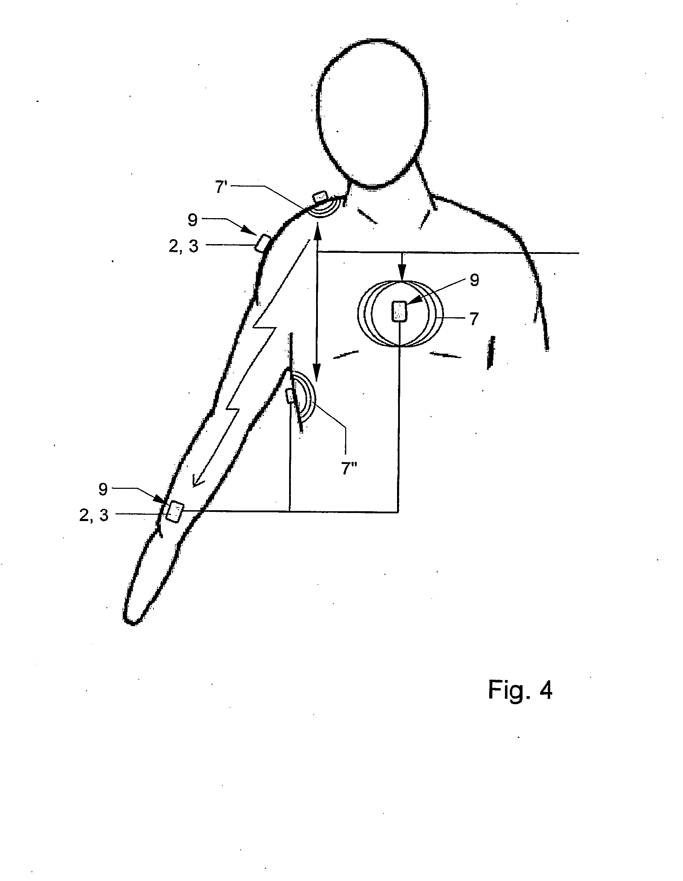

Fig. 4 shows a still further embodiment where, on at least a one, or more, body parts, both a transmitter 7 and a sensor cluster 9 (magnetic sensor module 2 and inertial measurement unit 3) are provided. Due to the fact that they have a fixed distance relationship with respect to one another, information coming from this receiver can be utilized for calibration and correction purposes of the measured values obtained from other sensor clusters 9 not positioned rigidly with respect to the transmitter 7. Therefore, in this configuration, the receivers are placed on different body parts, while, each time, a magnetic sensor 2 and inertial measurement unit 3 are placed on a same segment as the transmitter 7, while, incidentally, the sensor 2 may also be placed in another place relative to the transmitter 7, or not on the same segment. In the embodiments shown, the transmitters 7 are three single-axis coils, placed on one segment (the trunk 6) but in different positions, which essentially form a 3D whole in interrelationship. Other configurations are of course possible of 1, 2 or 3D transmitters, multiple of them placed in different positions on the body while a redundancy is realized by generating fields in more than three mutually different spatial directions. Further, an advantage is thus realized that the transmitters need to be less powerful because they can be adjusted to a smaller range, for instance only a lower part of the body or only an upper part of the body. A further advantage of the distributed transmitter configuration is that local disturbances of the magnetic field can thus be minimized due to a relatively greater field strength of the transmitter placed at a short distance. In particular, it should be kept in mind that the field strength of a for example a magnetic coil transmitter can be modeled as a dipole field having a fall-off characteristic of 1/d².

In particular, the relatively low control frequency of the transmitters enables the use of a large number of time slots, and consequently also a large number of distributed transmitters without interference occurring between the different transmitters. Here, the short range of the transmitters can also be utilized so that transmitters placed at a particular distance from one another can be controlled independently of one another.

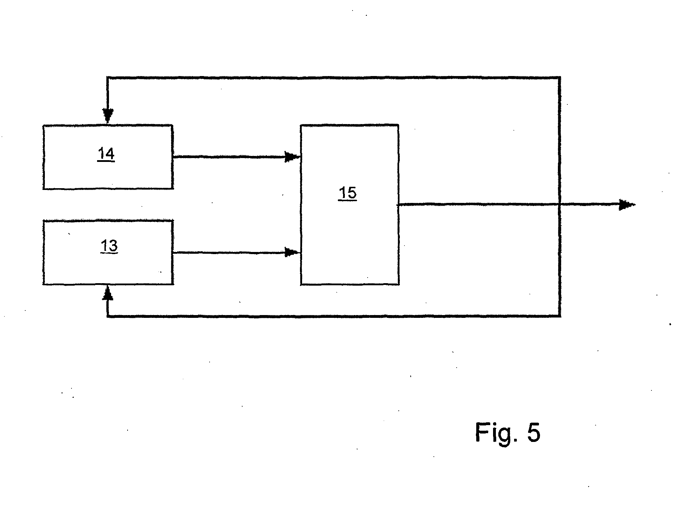

The Figures Fig. 5- Fig. 7 schematically show how the signal processing of the motion tracking system according to the invention is organized. A first step is periodically combining the data coming from the magnetic tracking system 13 and the data, in particular the measured angular velocities and linear accelerations, coming from the inertial measuring system 14. These data are combined in module 15 for deriving position and orientation information of the constituent object parts of the object on the basis of the received signals.

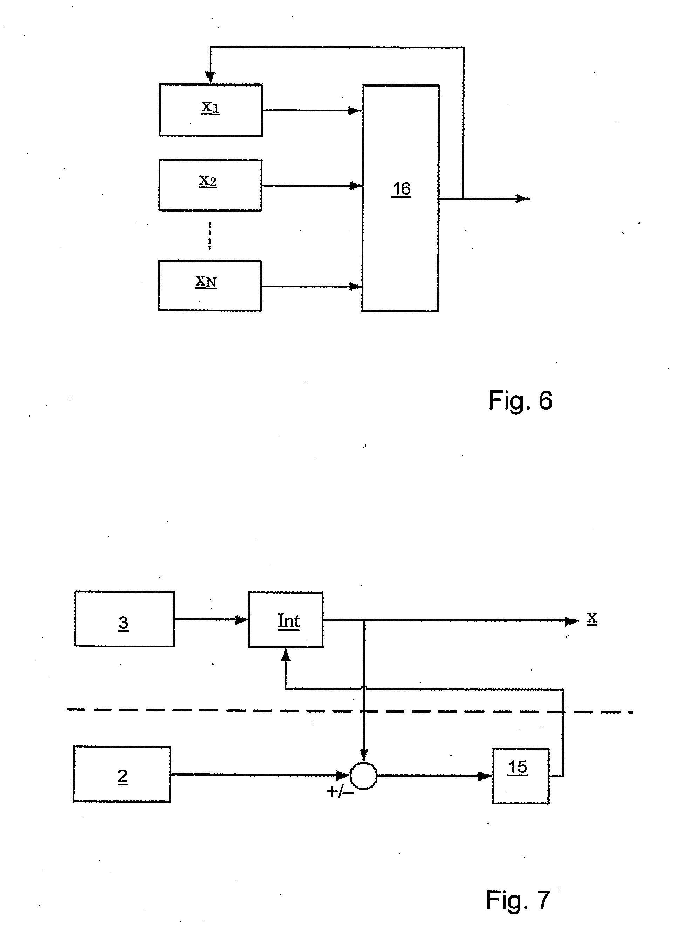

In Fig. 6, in a second module 16, the thus obtained position and orientation data of a single sensor are combined with the data of the other sensors, in combination with a feedback loop containing information about parameters of a model of the error of the sensor module. From this, a definitive estimation of the motion, in particular the position and orientation of the different measured body segments, of the body is made. Both modules 15, 16 advantageously make use of so-called Kalman filter methods. From this, also due to overdetermination of the measurements, the instantaneous measuring errors, in particular system and model errors, can be estimated. Errors in the body model may, for instance, be: the relationship in orientation and position between the sensor and the segment, the length of segments in the body model, the position of points of rotation between segments and parameters of a model of motion artifacts of the sensor cluster on the body segments. Errors in the sensor model may, for instance, be: magnitude of amplification factor (gain), offset, temperature sensitivity, and cross-sensitivity with other quantities.

Fig. 7, finally, shows the specific signal-processing structure according to the invention. Here, a distinction is made between determining motion data with a high time resolution with a relatively high error due to drift and integration errors of the inertial measurement unit 3 and determining motion data with the aid of the magnetic field sensors 2.

With the aid of magnetic field sensors, a calibration is used intermittently. To this end, the values coming from the magnetic field sensors and the inertial measurement unit, including their estimated measuring errors, are, for instance by means of weighing in a Kalman filter 15, fed back to the integration values of the inertial measurement units, so that an improved accuracy of position and orientation measurement is realized. On the basis of a derived measuring error, a new cycle of position measurement by means of magnetic fields can be activated.

Fig. 8 shows a preferred embodiment of the sensor clusters 9. On a common fixing platform 17, three sensors are fixed, each having a predetermined fixed 3D orientation with respect to one another and the fixing platform 17. The sensors comprise an inertial measurement unit 3 and a magnetic measuring module 2. The inertial measurement unit 3 comprises a linear accelerometer 18 and an angular velocity sensor 19. The linear accelerometer 18 measures an acceleration value *̅a̅*̅, in particular the values aₓ, a_{y} and a_{z} in the coordinate system of the accelerator, which has a predetermined orientation with respect to the fixing platform 17, which does not yield an actual acceleration after subtraction of the acceleration due to gravity *̅g̅*̅. The angular velocity meter 19 serves *inter alia* to be able to calculate this actual *̅a̅*̅-*̅g̅*̅ after an orientation correction. To this end, the angular velocity meter 19 outputs the measured angular velocity values ωₓ, ω_{y} and ω_{z} and also has a predetermined orientation with respect to the fixing platform 17.

It is well-known to those skilled in the art that these values can also be obtained in another manner, for instance by means of an inertial measurement unit where an angular acceleration is measured, which (integral) value is combined with the results of one, two or three-dimensional measured values of a linear acceleration transducer in a suitable manner. It is also conceivable that the required linear and angular acceleration values are derived from a single measuring sensor.

When the magnetic field transmitters 7 are not active, the magnetic measuring module 2 can measure the earth magnetic field B, which yields a fixed orientation. The magnetic measuring module 2 also has a predetermined orientation and position with respect to the fixing platform 17. Upon activating the magnetic field transmitters 7, the magnetic module can record an orientation and position value of the module 2 with respect to the field transmitter 7, so that a relative position and/or orientation of the module 2 with respect to the transmitter 7 is measured by reading out the measuring values mₓ, m_{y}, en m_{z}. This value can then be combined with the position and orientation values measured from the inertial module to (re)calibrate the measured values of the inertial module as discussed hereinabove.

Although, in the specification, the invention is explained with reference to motion tracking of persons, other objects may also be tracked. In particular, what this system offers is measuring the relative distance and orientation of objects standing separately with respect to one another, which together form one moving system in the space. Options to be considered here are robot control, etc. These variations are understood to fall within the scope of the claims as defined in the following.

## Claims

1. A motion tracking system for tracking an object composed of object parts in a three-dimensional space, comprising:
- a transmitter module (7) adapted to be provided in fixed positional relationship with respect to at least one object part (1), for transmitting magnetic fields, with mutually different main directions;
- a magnetic measuring module (2) for receiving the magnetic fields, wherein the magnetic measuring module is adapted to have a fixed positional relationship with an object part of the composite object; **characterized in**
- an inertial measurement unit (3) for recording a linear acceleration and an angular velocity or angular acceleration, wherein the inertial measurement unit is adapted to have a fixed positional relationship with an object part of the composite object;
- a processor (10) for controlling the transmitter module (7) and receiving signals coming from the magnetic measuring module and the inertial measurement unit; which processor contains a module for deriving orientation and/or position information of the constituent object parts of the object on the basis of the received signals,
- the processor (10) being configured to make the transmitters (7) transmit an intermittent magnetic field, wherein the position and/or orientation information in a period between the intermittent transmitting is derived by means of the motion information coming from the inertial measurement unit (3), which is periodically calibrated with the motion information coming from the magnetic measuring module (2).

2. A motion tracking system according to claim 1, wherein the period between the intermittent transmitting is longer than a predetermined period related to a motion resolution realized by the system.

3. A motion tracking system according to claim 1, wherein the period between the intermittent transmitting is longer than 0.08 seconds.

4. A motion tracking system according to at least one of the preceding claims, **characterized in that** the processor (10) contains a module for combining position and/or orientation information of an object part on the basis of the received signals with respect to at least one fixed axis in the space which is derived from the gravitational field (g) and/or the earth magnetic field.

5. A motion tracking system according to at least one of the preceding claims, **characterized in that** a number of sensors are provided for fixing to the object for measuring a reaction force and/or torque.

6. A motion tracking system according to at least one of the preceding claims, wherein the module (15, 16) for combining position and/or orientation information comprises a Kalman filter.

7. A motion tracking system according to at least one of the preceding claims, wherein the module (15,16) is arranged for deriving a measuring error of the position and/or orientation information.

8. A motion tracking system according to at least one of the preceding claims, wherein, on the basis of a derived measuring error, a new cycle of orientation and/or position measurement by means of magnetic fields is activated.

9. A motion tracking system according to at least one of the preceding claims, wherein a receiver is arranged to be provided in a fixed positional relationship with respect to a transmitter for calibration purposes.

10. A motion tracking system according to at least one of the preceding claims, wherein more than four transmitters are provided on the object in a distributed manner.

11. A motion tracking system according to at least one of the preceding claims, wherein the object is a human body wherein the transmitters are integrated in clothing.

12. A motion tracking system according to claim 11, wherein a transmitter comprises at least one current coil for generating a magnetic field, which current coil is intended for enclosing a body part.

13. A motion tracking system according to at least one of the preceding claims, wherein the module for combining orientation and/or position information of the constituent object parts comprises a model of the freedom of movement of the object parts relative to one another and estimates the measuring signals with respect to that model.

14. A motion tracking system according to at least one of the preceding claims, wherein a time order is monitored between the transmitters by means of time slots.

15. A motion tracking system according to at least one of the preceding claims, wherein the transmitters each generate an oriented magnetic field by means of a current coil.

16. A motion tracking system according to at least one of the preceding claims, wherein the magnetic measuring module and the inertial measurement unit are included in one or more sensor clusters for fixing on the object.

## Patentansprüche

1. Bewegungsverfolgungssystem zum Verfolgen eines Objekts, das aus Objektteilen in einem dreidimensionalen Raum besteht, mit folgenden Merkmalen:
- einem Sendermodul (7), das dazu ausgebildet ist, in einer festen Positionsbeziehung zu mindestens einem Objektteil (1) vorgesehen zu werden, um Magnetfelder mit zueinander verschiedenen Hauptrichtungen auszusenden;
- einem Magnetmessmodul (2) zum Empfangen der Magnetfelder, wobei das Magnetmessmodul dazu ausgebildet ist, eine feste Positionsbeziehung zu einem Objektteil des zusammengesetzten Objektes zu haben;
**dadurch gekennzeichnet,**
- **dass** eine Trägheitsmesseinheit (3) zum Aufzeichnen einer linearen Beschleunigung sowie einer Winkelgeschwindigkeit oder Winkelbeschleunigung vorgesehen ist, wobei die Trägheitsmesseinheit dazu ausgebildet ist, eine feste Positionsbeziehung zu einem Objektteil des zusammengesetzten Objektes zu haben;
- **dass** ein Prozessor (10) vorgesehen ist zum Steuern des Sendermoduls (7) und zum Empfangen von Signalen, die von dem Magnetmessmodul und der Trägheitsmesseinheit kommen, wobei der Prozessor ein Modul zum Ableiten von Orientierungs- und/oder Positionsinformation der Objektbestandteile des Objektes auf der Basis der empfangenen Signale enthält; und
- **dass** der Prozessor (10) derart konfiguriert ist, dass die Sender (7) ein intermittierendes Magnetfeld aussenden, wobei die Positions- und/oder Orientierungsinformation in einer Periode zwischen den intermittierenden Sendevorgängen mittels der Bewegungsinformation von der Trägheitsmesseinheit (3) abgeleitet wird, die periodisch mit der Bewegungsinformation kalibriert wird, die von dem Magnetmessmodul (2) kommt.

2. Bewegungsverfolgungssystem nach Anspruch 1, **dadurch gekennzeichnet, dass** die Periode zwischen den intermittierenden Sendevorgängen länger als eine vorbestimmte Periode ist, die zu einer realisierten Bewegungsauflösung des Systems in Beziehung steht.

3. Bewegungsverfolgungssystem nach Anspruch 1, **dadurch gekennzeichnet, dass** die Periode zwischen den intermittierenden Sendevorgängen länger als 0,08 Sekunden ist.

4. Bewegungsverfolgungssystem nach mindestens einem der vorhergehenden Ansprüche, **dadurch gekennzeichnet, dass** der Prozessor (10) ein Modul enthält zum Kombinieren von Positions- und/oder Orientierungsinformation eines Objektteils auf der Basis der empfangenen Signale gegenüber mindestens einer festen Achse im Raum, die aus dem Gravitationsfeld (g) und/oder dem Erdmagnetfeld abgeleitet ist.

5. Bewegungsverfolgungssystem nach mindestens einem der vorhergehenden Ansprüche, **dadurch gekennzeichnet, dass** eine Anzahl von Sensoren zum Befestigen an dem Objekt vorgesehen ist zum Messen einer Reaktionskraft und/oder eines Reaktionsmomentes.

6. Bewegungsverfolgungssystem nach mindestens einem der vorhergehenden Ansprüche, **dadurch gekennzeichnet, dass** das Modul (15, 16) zum Kombinieren der Positions- und/oder Orientierungsinformation ein Kalman-Filter enthält.

7. Bewegungsverfolgungssystem nach mindestens einem der vorhergehenden Ansprüche, **dadurch gekennzeichnet, dass** das Modul (15, 16) vorgesehen ist zum Ableiten eines Messfehlers der Positions- und/oder Orientierungsinformation.

8. Bewegungsverfolgungssystem nach mindestens einem der vorhergehenden Ansprüche, **dadurch gekennzeichnet, dass** auf der Basis eines abgeleiteten Messfehlers ein neuer Zyklus einer Orientierungs- und/oder Positionsmessung mittels Magnetfelder aktiviert wird.

9. Bewegungsverfolgungssystem nach mindestens einem der vorhergehenden Ansprüche, **dadurch gekennzeichnet, dass** ein Empfänger für Kalibrationszwecke in einer festen Positionsbeziehung zu dem Sender vorgesehen ist.

10. Bewegungsverfolgungssystem nach mindestens einem der vorhergehenden Ansprüche, **dadurch gekennzeichnet, dass** mehr als vier Sender an einem Objekt in verteilter Weise vorgesehen sind.

11. Bewegungsverfolgungssystem nach mindestens einem der vorhergehenden Ansprüche, **dadurch gekennzeichnet, dass** das Objekt ein menschlicher Körper ist und die Sender in die Kleidung integriert sind.

12. Bewegungsverfolgungssystem nach Anspruch 11, **dadurch gekennzeichnet, dass** ein Sender mindestens eine Stromspule zum Erzeugen eines Magnetfeldes enthält, wobei die Stromspule dazu gedacht ist, ein Körperteil zu umschließen.

13. Bewegungsverfolgungssystem nach mindestens einem der vorhergehenden Ansprüche, **dadurch gekennzeichnet, dass** das Modul zum Kombinieren von Orientierungs- und/oder Positionsinformation der Objektbestandteile ein Modell für die Freiheit der Bewegung der Objektteile relativ zueinander enthält und dass es die Messsignale mit Bezug auf dieses Modell berechnet.

14. Bewegungsverfolgungssystem nach mindestens einem der vorhergehenden Ansprüche, **dadurch gekennzeichnet, dass** eine Zeitreihenfolge zwischen den Sendern anhand von Zeitschlitzen angezeigt wird.

15. Bewegungsverfolgungssystem nach mindestens einem der vorhergehenden Ansprüche, **dadurch gekennzeichnet, dass** jeder der Sender ein orientiertes Magnetfeld mittels einer Stromspule erzeugt.

16. Bewegungsverfolgungssystem nach mindestens einem der vorhergehenden Ansprüche, **dadurch gekennzeichnet, dass** das Magnetmessmodul und die Trägheitsmesseinheit in einem oder mehreren Sensorbündeln zum Befestigen an dem Objekt enthalten sind.

## Revendications

1. Système de suivi de mouvement pour suivre un objet constitué de parties d'objet dans un espace tridimensionnel, comprenant :
- un module émetteur (7) adapté pour être aménagé en relation de position fixe par rapport à au moins une partie d'objet (1), pour transmettre des champs magnétiques, avec des directions principales mutuellement différentes ;
- un module de mesure magnétique (2) pour recevoir les champs magnétiques, le module de mesure magnétique étant adapté pour avoir une relation de position fixe avec une partie de l'objet composite ;
**caractérisé par**
- une unité de mesure inertielle (3) pour enregistrer une accélération linéaire et une vitesse angulaire ou une accélération angulaire, l'unité de mesure inertielle étant adaptée pour avoir une relation de position fixe avec une partie de l'objet composite ;
- un processeur (10) pour commander le module émetteur (7) et recevoir des signaux provenant du module de mesure magnétique et de l'unité de mesure inertielle ; lequel processeur contient un module pour déduire des informations d'orientation et/ou de position des parties d'objet constitutives de l'objet sur la base des signaux reçus,
- le processeur (10) étant configuré pour permettre aux émetteurs (7) de transmettre un champ magnétique intermittent, dans lequel les informations de position et/ou d'orientation situées dans une période entre des émissions intermittentes sont déduites au moyen des informations de mouvement provenant de l'unité de mesure inertielle (3), qui est étalonnée périodiquement avec les informations de mouvement provenant du module de mesure magnétique (2).

2. Système de suivi de mouvement selon la revendication 1, dans lequel la période entre les émissions intermittentes est supérieure à une période prédéterminée rapportée à une résolution de mouvement réalisé par le système.

3. Système de suivi de mouvement selon la revendication 1, dans lequel la période entre les émissions intermittentes est supérieure à 0,08 seconde.

4. Système de suivi de mouvement selon au moins l'une quelconque des revendications précédentes, **caractérisé en ce que** le processeur (10) contient un module pour combiner des informations de position et/ou d'orientation d'une partie d'objet sur la base des signaux reçus par rapport à au moins un axe fixe de l'espace, qui est tiré du champ gravitationnel (g) et/ou du champ magnétique terrestre.

5. Système de suivi de mouvement selon au moins l'une quelconque des revendications précédentes, **caractérisé en ce qu'**il est prévu un certain nombre de capteurs pour fixer à l'objet dans le but de mesurer une force de réaction et/ou un couple de torsion.

6. Système de suivi de mouvement selon au moins l'une quelconque des revendications précédentes, dans lequel le module (15, 16) pour combiner des informations de position et/ou d'orientation comprend un filtre de Kalman.

7. Système de suivi de mouvement selon au moins l'une quelconque des revendications précédentes, dans lequel le module (15, 16) est aménagé pour déduire une erreur de mesure des informations de position et/ou d'orientation.

8. Système de suivi de mouvement selon au moins l'une quelconque des revendications précédentes, dans lequel, sur la base d'une erreur de mesure déduite, un nouveau cycle de mesure d'orientation et/ou de position au moyen de champs magnétiques est activé.

9. Système de suivi de mouvement selon au moins l'une quelconque des revendications précédentes, dans lequel un récepteur est aménagé pour se trouver dans une relation de position fixe par rapport à un émetteur à des fins d'étalonnage.

10. Système de suivi de mouvement selon au moins l'une quelconque des revendications précédentes, dans lequel plus de quatre émetteurs sont aménagés sur l'objet de manière distribuée.

11. Système de suivi de mouvement selon au moins l'une quelconque des revendications précédentes, dans lequel l'objet est un corps humain, les émetteurs étant intégrés aux vêtements.

12. Système de suivi de mouvement selon la revendication 11, dans lequel un émetteur comprend au moins une bobine de courant pour générer un champ magnétique, laquelle bobine de courant est destinée à enserrer une partie du corps.

13. Système de suivi de mouvement selon au moins l'une quelconque des revendications précédentes, dans lequel le module pour combiner des informations d'orientation et/ou de position des parties d'objet constitutives comprend un modèle de liberté de mouvement des parties d'objet l'une par rapport à l'autre, et estime les signaux de mesure par rapport à ce modèle.

14. Système de suivi de mouvement selon au moins l'une quelconque des revendications précédentes, dans lequel une chronologie est contrôlée entre les émetteurs au moyen de créneaux de temps.

15. Système de suivi de mouvement selon au moins l'une quelconque des revendications précédentes, dans lequel les émetteurs génèrent chacun un champ magnétique orienté au moyen d'une bobine de courant.

16. Système de suivi de mouvement selon au moins l'une quelconque des revendications précédentes, dans lequel le module de mesure magnétique et l'unité de mesure inertielle sont inclus dans un ou plusieurs groupes de capteurs pour fixation sur l'objet.
